# EUROPEAN PATENT SPECIFICATION

(11) **EP 3 479 793 B1**
(45) Date of publication and mention of the grant of the patent: **16.12.2020**
(21) Application number: 17820096.0
(22) Date of filing: 26.06.2017
(51) Int. Cl.: A61C 5/40, B65D 35/02, B65D 35/08, B65D 77/20, A61C 5/66

(54) **VISCOUS DENTAL MATERIAL PACKAGE**
VERPACKUNG FÜR VISKOSE DENTALMATERIALIEN
EMBALLAGE POUR MATÉRIAUX DENTAIRE VISQUEUX

(30) Priority: 30.06.2016 JP 2016130504
(43) Date of publication of application: 08.05.2019
(73) Proprietor: GC Corporation, Sunto-gun, Shizuoka 410-1307 (JP)
(72) Inventor: SUZUKI, Takumi, Tokyo 174-8585 (JP); FUJIMAKI, Yuji, Tokyo 174-8585 (JP)
(74) Representative: Müller-Boré & Partner Patentanwälte PartG mbB
(86) International application number: PCT/JP2017/023405
(87) International publication number: WO 2018/003741

(56) References cited:
- WO-A1-2008/093596
- WO-A1-2008/093596
- JP-A- H0 911 416
- JP-A- H0 911 416
- JP-A- 2005 119 665
- JP-A- 2005 119 665
- JP-A- 2009 106 654
- JP-A- 2009 106 654
- JP-A- 2013 216 599
- US-A- 1 592 584
- US-A- 2 430 995

## Description

### Technical Field

The present invention relates to a packaging body for viscous dental material that includes a container and a viscous dental material filled in the container.

### Background Art

When viscous dental materials, such as dental filling materials, materials for artificial teeth, and materials for producing dental crown materials, are treated in the dental field, such viscous dental materials of high viscosity are commonly provided in a mode of being filled in a cylindrical disposable container (packaging body for viscous dental material).

Structures and use modes of common packaging bodies for viscous dental material are disclosed for example in Patent Literatures 1 to 4. That is, a pressing member (e.g. piston to be screwed with threads) is inserted into a cylindrical member (e.g. syringe) with a viscous dental material in it from one end of the cylindrical member, and a small amount of the viscous dental material is extruded from the other end of the cylindrical member. Then, the extruded viscous dental material is scraped with a dental instrument of stick shape having a spherical or spatular end, and applied to a tooth to be healed or a dental material to be produced.

### Citation List

### Patent Literature

Patent Literature 1: JPH06-20468 B
Patent Literature 2: JPH08-56962 A
Patent Literature 3: JP2006-61739 A
Patent Literature 4: JP3102378 U
Patent Literature 5: JPH0911416 A describes equipping a layer produced by adding an amorphous or low scrystalline copolymer of an olefin and a cyclic olefin as an intermediate layer and providing a multilayered container excellent in aromaproofness, water retention and contents resistance and further laminar bondability, especially a crushable multilayered container. This multilayered container is equipped with an inner and outer layers 11 and 13, which contain an olefin-based resin, and an intermediate layer 12, which contains an amorphous or low crystalline copolymer of an olefin and cyclic olefin.
Patent Literature 6: US 2 430 995 A describes a collapsable container storing a pharmaceutical material, wherein the container has a tubular shape and is produced by extrusion molding, and wherein both of its ends are sealed by joining the edges on each end together.

### Summary of Invention

### Technical Problem

The techniques described in these Patent Literatures have effects relating to extraction of viscous dental materials. However, each structure thereof tends to be complicated and bulky, which causes problems in view of productivity, price, amount of wastes when disposed of, and the like.

An object of the present invention is to provide a packaging body for viscous dental material of easy structure.

### Solution to Problem

Hereinafter the present invention will be described. Here, reference numerals added to the drawings are also described in parentheses for easy understanding. However, the present invention is not limited to them.

An aspect of the present invention is a packaging body for viscous dental material comprising: a container of cylindrical shape whose wall thickness is 0.1 mm to 0.8 mm, including one end closed with a sealed part that is formed of facing edges of the one end, the edges being joined together; and a viscous dental material filled inside the container, having a consistency of 10 mm to 25 mm.

### Advantageous Effects of Invention

According to the present invention, the packaging body for viscous dental material has very easy structure, and the amount of wastes after use of the viscous dental material can be decreased.

### Brief Description of Drawings

FIG. 1A is a plan view of a packaging body for viscous dental material 10, FIG. 1B is a side view of the packaging body for viscous dental material 10, FIG. 1C is a cross-sectional view taken along Ic-Ic in the packaging body for viscous dental material 10, and
FIG. 1D is a cross-sectional view taken along Id-Id in the packaging body for viscous dental material 10;
FIGs. 2A to 2C are views to explain a situation where the packaging body for viscous dental material 10 is used;
FIG. 3A is a view to explain a packaging body for viscous dental material 20, and FIG. 3B (this embodiment is not covered by claim 1) is a view to explain a packaging body for viscous dental material 30; and
FIG. 4A is a plan view of a packaging body for viscous dental material 40, and FIG. 4B is a side view of the packaging body for viscous dental material 40.

### Description of Embodiments

Hereinafter the present invention will be explained based on the embodiments shown in the drawings. However, the present invention is not limited to the embodiments.

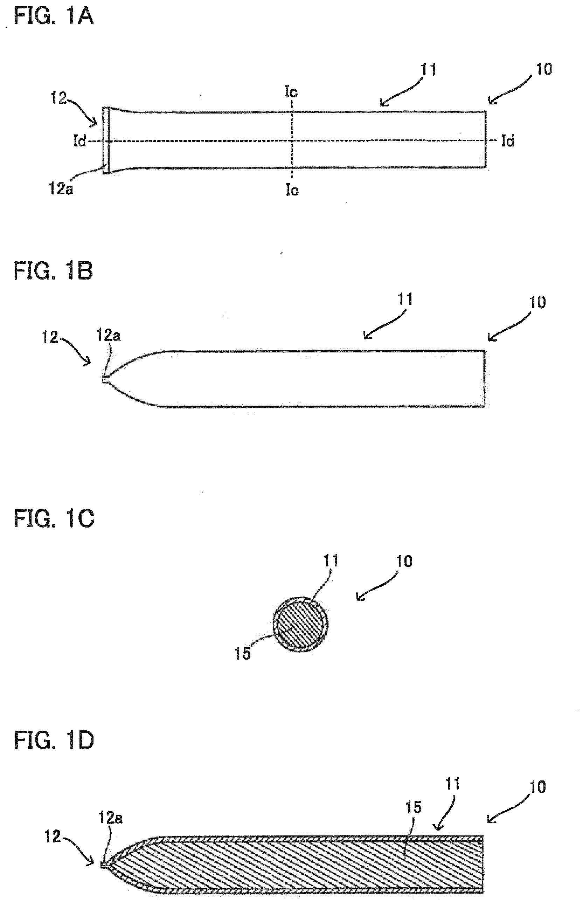

FIG. 1 includes views to explain an embodiment, explaining a packaging body for viscous dental material 10. FIG. 1A is a plan view, FIG. 1B is a side view, FIG. 1C is a Ic-Ic cross-sectional view, and FIG. 1D is a Id-Id cross-sectional view.

As seen from FIG. 1, the packaging body for viscous dental material 10 of this embodiment includes a container 11 and a viscous dental material 15.

The container 11 is a container in which the viscous dental material 15 is to be filled, and is cylindrical in shape, whose one end is a sealed end 12 that is closed by a sealed part 12a. In this embodiment, the other end is open, which allows, for example, insertion of instruments into the container 11.

The sealed part 12a is formed of facing edges of the one end on the sealed end 12 side of the cylindrical shape. The edges are squashed to face to each other and joined together. Thus, an opening of the container 11 can be closed without any other members, resulting in a very easy structure.

The size etc. of the container 11 is not limited to particular values. The thickness of the container 11 is preferably 0.1 mm to 0.8 mm. If the thickness is smaller than 0.1 mm, the container 11 easily deforms, and defects such as incorporation of air bubbles easily occurs. If the thickness is over 0.8 mm, the container 11 becomes very hard and difficult to cut with scissors and the like.

The material of the container 11 is not particularly limited, and a hard material having a predetermined strength is preferable. Specific preferable examples include polyethylene, polyacetal, polypropylene, polyamide, vinyl chloride resin, nylon, phenol resin, polyurethane, saturated polyester resin, melamine resin, polyvinylidene chloride, unsaturated polyester resin, polybutadiene, polystyrene, EVA (ethylene-vinyl acetate copolymer) resin, styrol resin, polymethylpentene, methacrylic styrene, ABS (acrylonitrile, butadiene, styrene) resin, polycarbonate resin, and the like.

The container 11 is produced by extrusion molding.

The container 11 formed of a comparatively hard material can be squashed to a large degree by force from a person and the like, and can prevent incorporation of air bubbles into the viscous dental material when the container is squashed or recovers its shape.

On the other hand, the container 11 may be cut with scissors and the like with a decrease in the amount of the viscous dental material as it is used. Thus, it is preferable to hold down the hardness of the container 11 to such a degree that the container 11 can be cut.

The viscous dental material 15 is for example a dental filling material, a material for artificial tooth, and a material for producing dental crown materials in the dental field. Specific examples thereof include a paste material called composite resin, which is a curable resin that has a color of teeth and cures when irradiated with light. The base material of the composite resin is a multifunctional monomer, and fillers such as glass powder and silica powder are dispersed in the resin.

The viscous dental material 15 is a material of high viscosity, among the above-described materials. Its specific viscosity is of 10 mm to 25 mm of consistency. If the consistency is less than 10 mm, the material is too hard and is basically unfavorable for treatment. If the consistency is over 25 mm, which means the viscosity is too low, the viscous dental material may leak from the packaging body for viscous dental material, or is difficult to be scraped and used with dental instruments such as spatula. Preferably, the consistency is 10 mm to 15 mm.

Here, the consistency is defined by a value measured by the following method. That is: a sample formed by vacuum-defoaming a prepared viscous dental material and thereafter letting the obtained material stand at 25°C for 2 hours is used; the sample was weighed to be 0.5 mL, allowed to stand on a glass plate (5 cm × 5 cm) in a thermostatic chamber at 25°C (40% of humidity), in a manner to be heaped at a center of the glass plate; then, on the sample, a 40 g glass plate (5 cm × 5 cm) is put; thereafter, the long diameter and the short diameter of the sample after 120 seconds has passed is measured through the glass plate; the consistency is defined as a calculated value of the arithmetic average of the long diameter and the short diameter. The long diameter is the longest diameter of the sample among the diameters that pass the center of the sample, and the short diameter is the diameter that is orthogonal to the long diameter among the diameters that pass the center of the sample.

Such a viscous dental material 15 is filled inside the container 11 of cylindrical shape.

The packaging body for viscous dental material 10 described above may be produced as below for example.

That is, firstly, a cylindrical body to be the container 11, whose both ends are open, is prepared.

Next, the viscous dental material 15 is filled inside the cylindrical body, from one open end.

Then, one open end of the cylindrical shape is squashed and the edge of the squashed open end is joined by heat welding to form the sealed part 12a, to be the sealed end 12.

According to such a production method, a viscous dental material can be filled inside the container 11 without incorporation of air bubbles into the viscous dental material.

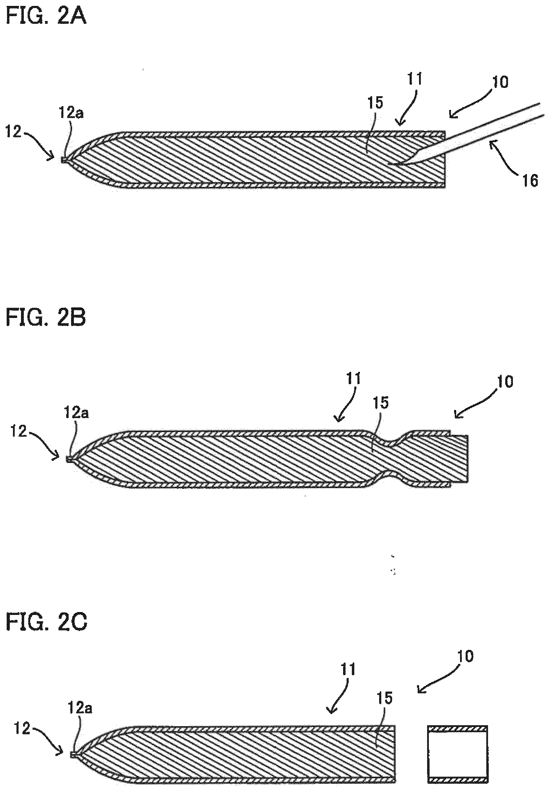

The packaging body for viscous dental material 10 described above is used as below for example. FIG. 2 includes views for explanation. FIGs. 2A to 2C are cross-sectional views showing a situation where the packaging body for viscous dental material 10 is used, as an example.

As shown by FIG. 2A, a practitioner such as dentist inserts a dental instrument 16 of stick shape having a spherical or spatular end into the container 11 from the open end of the container 11, scrapes the viscous dental material 15, and then applies the viscous dental material 15 to a tooth to be healed or a surface of a dental material to be produced.

Or, as shown by FIG. 2B, a practitioner such as dentist pushes the outer periphery of the container 11, to push out part of the viscous dental material 15 from the open end of the container 11. Then, the practitioner scrapes the viscous dental material 15 pushed out from the open end with a dental instrument, and applies it to a tooth to be healed or a surface of a dental material to be produced.

Repeating such scrape causes the viscous dental material 15 to decrease, and at the open end side of the container 11, the viscous dental material 15 is gone and only the container 11 is left. Then, the portion of the container 11 where the viscous dental material 15 is gone is cut with scissors and the like, as shown by FIG. 2C. This makes it possible to scrape the viscous dental material 15 again as described above, with the dental instrument 16.

The packaging body for viscous dental material 10 described above has an easy structure and excellent productivity and price. In addition, the user can reduce wastes when disposing of it after use.

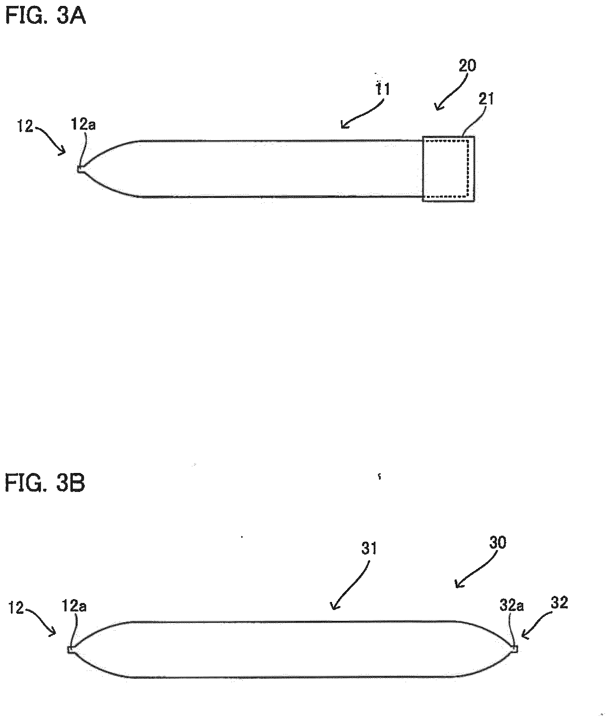

FIG 3A is a view to explain a packaging body for viscous dental material 20 according to a modification, and FIG. 3B (this embodiment is not covered by claim 1) is a view to explain a packaging body for viscous dental material 30 according to a modification.

As can be seen from FIG. 3A, the packaging body for viscous dental material 20 comprises the above-described packaging body for viscous dental material 10 and a lid 21 attachable to the open end of the packaging body for viscous dental material 10. This makes it possible to avoid dirt and polymerization to cure of the viscous dental material 15 by exposure to light, when the viscous dental material 15 is not used, in addition to the above effect of the packaging body for viscous dental material 10.

As can be seen from FIG. 3B (this embodiment is not covered by claim 1), the packaging body for viscous dental material 30 is an example where the both ends of a container 31 are respectively provided with sealed parts 12a and 32a, to be sealed ends 12 and 32. In addition to the above effect of the packaging body for viscous dental material 10, the packaging body for viscous dental material 30 can improve the quality maintenance during transportation and storage after production, because the both ends are sealed until used. Then, the sealed part 32 is cut by scissors and the like in use.

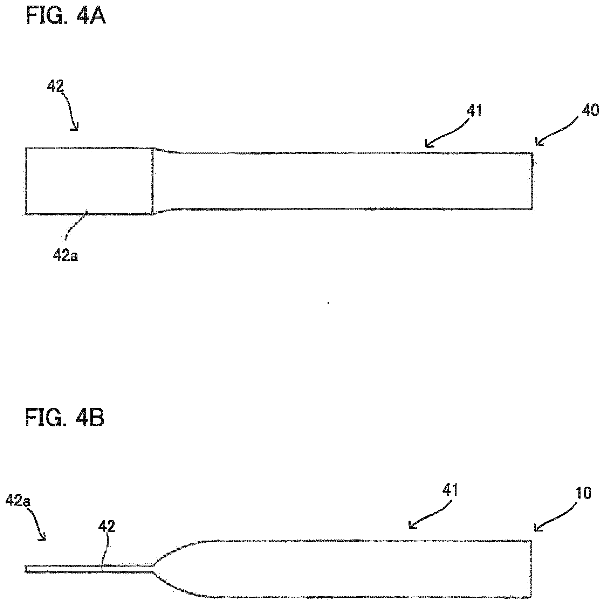

FIG. 4 includes views to explain a packaging body for viscous dental material 40 according to another modification. FIG. 4A is a plan view of the packaging body for viscous dental material 40, and FIG. 4B is a side view of the packaging body for viscous dental material 40.

In this embodiment, a sealed part 42a at a sealed end 42 is formed in a manner to extend long in a direction of an axis of the container 11. This makes it possible to use the sealed part 42a as a handle or a space to attach labels, which increases the usability.

### Reference Signs List

- 10, 20, 30, 40: packaging body for viscous dental material
- 11, 31, 41: container
- 12, 32, 42: sealed end
- 12a, 32a, 42a: sealed part
- 15: viscous dental material
- 21: lid

## Claims

1. A packaging body for viscous dental material (10), the body including a container (11), and a viscous dental material (15) filling the container, wherein the container (11) is produced by extrusion molding to have a cylindrical shape whose wall thickness is 0.1 mm to 0.8 mm, including one end closed with a sealed part that is formed of facing edges of the one end, the edges being joined together, and another end opposite the sealed part, the other end being open to allow an instrument to be inserted in the container; and wherein the viscous dental material (15) inside the container has a consistency of 10 mm to 25 mm, the consistency being obtained by: vacuum-defoaming the material (15), which is prepared, and thereafter letting the material stand at 25°C for 2 hours; weighing 0.5 mL of the obtained material to allow the weighed material to stand on a glass plate at 25°C and 40% of humidity in such a manner to be heaped at the glass plate; and putting a 40 g glass plate on the weighed material to measure a long diameter and a short diameter of the weighed material through the glass plate after 120 seconds has passed, to calculate an arithmetic average of the long diameter and the short diameter.

## Patentansprüche

1. Ein Verpackungskörper für zähflüssiges Dentalmaterial (10), wobei der Körper einen Behälter (11) beinhaltet und ein zähflüssiges Dentalmaterial (15) den Behälter füllt,
wobei der Behälter (11) durch Extrusionsformen hergestellt ist, sodass er eine zylindrische Form hat, deren Wanddicke 0,1 mm bis 0,8 mm beträgt, wobei er Folgendes beinhaltet: ein Ende, das mit einem abgedichteten Teil verschlossen ist, das aus einander gegenüberliegenden Rändern des einen Endes gebildet ist, wobei die Ränder miteinander verbunden sind, und ein weiteres Ende gegenüber dem abgedichteten Teil, wobei das andere Ende offen ist, um das Einführen eines Instruments in den Behälter zu ermöglichen; und
wobei das zähflüssige Dentalmaterial (15) im Inneren des Behälters eine Konsistenz von 10 mm bis 25 mm hat, wobei die Konsistenz erhalten wird durch: Vakuum-Entschäumen des Materials (15), das vorbereitet wird, und anschließendes Stehenlassen des Materials bei 25 °C für 2 Stunden; Wiegen von 0,5 mL des erhaltenen Materials, um das gewogene Material auf einer Glasplatte bei 25 °C und 40% Feuchtigkeit so stehen zu lassen, dass es auf die Glasplatte aufgehäuft wird; und Auflegen einer 40 g schweren Glasplatte auf das gewogene Material, um durch die Glasplatte einen langen Durchmesser und einen kurzen Durchmesser des gewogenen Materials zu messen, nachdem 120 Sekunden vergangen sind, um ein arithmetisches Mittel des langen Durchmessers und des kurzen Durchmessers zu berechnen.

## Revendications

1. Un corps d'emballage pour matériau dentaire visqueux (10), le corps incluant un récipient (11), et un matériau dentaire visqueux (15) remplissant le récipient,
sachant que le récipient (11) est produit par moulage par extrusion pour avoir une forme cylindrique dont l'épaisseur de paroi est de 0,1 mm à 0,8 mm, incluant une extrémité fermée par une partie scellée qui est constituée de bords se faisant face de cette extrémité, les bords étant joints ensemble, et une autre extrémité opposée à la partie scellée, l'autre extrémité étant ouverte pour permettre à un instrument d'être inséré dans le récipient ; et
sachant que le matériau dentaire visqueux (15) à l'intérieur du récipient présente une consistance de 10 mm à 25 mm, la consistance étant obtenue par le fait de : démousser sous vide le matériau (15), qui est préparé, et ensuite de laisser le matériau reposer à 25 °C pendant 2 heures ; peser 0,5 ml du matériau obtenu pour permettre au matériau pesé de reposer sur une plaque de verre à 25 °C et 40% d'humidité de manière à être entassé sur (at) la plaque de verre ; et de placer une plaque de verre de 40 g sur le matériau pesé pour mesurer un long diamètre et un court diamètre du matériau pesé à travers la plaque de verre après 120 secondes, afin de calculer une moyenne arithmétique du long diamètre et du court diamètre.
